(19) Europäisches Patentamt
European Patent Office
Office européen des brevets

(11) Veröffentlichungsnummer : **0 091 033**
**B1**

(12) **EUROPÄISCHE PATENTSCHRIFT**

(45) Veröffentlichungstag der Patentschrift : **26.06.85**

(51) Int. Cl.⁴ : **C 07 C 47/445**, C 07 C 49/553, C 07 C 45/69, C 11 B 9/00, A 61 K 7/46

(21) Anmeldenummer : **83102974.9**

(22) Anmeldetag : **25.03.83**

# (54) Trimethylbicyclo(4.3.0)non-1-en-Derivate, deren Herstellung und Verwendung als Riechstoffe, sowie diese enthaltende Riechstoffkompositionen.

(30) Priorität : **02.04.82 DE 3212326**

(43) Veröffentlichungstag der Anmeldung : **12.10.83 Patentblatt 83/41**

(45) Bekanntmachung des Hinweises auf die Patenterteilung : **26.06.85 Patentblatt 85/26**

(84) Benannte Vertragsstaaten :
**CH DE FR GB LI NL**

(56) Entgegenhaltungen :
**EP-A- 0 021 356**
**FR-A- 2 316 922**
**CHEMICAL ABSTRACTS, Band 88, Nr. 11, 13. März 1978, Seite 473, Nr. 74232r, Columbus, Ohio, US**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Bruns, Klaus, Dr.**
**Notburgaweg 6**
**D-4150 Krefeld-Traar (DE)**
Erfinder : **Weber, Ursula**
**Ratherstrasse 86**
**D-4150 Krefeld-Traar (DE)**

**EP 0 091 033 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Aus DE-OS-29 25 622 ist bekannt, daß das Isomerengemisch 4(5)-Acetyl-7,7,9(7,9,9)-trimethyl-bicyclo [4.3.0] non-1-en ein wertvoller Riechstoff mit warmem Ambraduft ist.

Es wurden nun weitere Trimethylbicyclo [4.3.0] non-1-en-Derivate gefunden, die ebenfalls wertvolle Riechstoffe mit interessanten und überraschend unterschiedlichen Duftnoten sind. Die Herstellung der neuen Verbindungen erfolgt nach an sich bekannten Syntheseverfahren der organischen Chemie. Als Ausgangsmaterial dient 2,2,4 [2,4,4]-Trimethylcyclopentanon (I), das stets als Isomerengemisch vorliegt und als einheitliche Verbindung nicht erhältlich ist. Dieses wird mittels Grignard-Reaktion zum 1-Vinyl-1-Hydroxy-2,2,4 [2,4,4]-trimethylcyclopentan (II) umgesetzt. Durch Dehydratisierung mittels p-Toluolsulfonsäure gelangt man zum 1-Vinyl-2,2,4 [2,4,4]-trimethylcyclopent-1-en (III), das jeweils aus 3 Isomeren besteht. Dieses wird durch Umsetzung mit geeigneten dienophilen Aldehyden oder Ketonen nach Diels-Alder in die neuen Trimethylbicyclo [4,3,0] non-1-en-Derivate (IV-IX) übergeführt, die als odophore Funktion Acyl- bzw. Aldehydgruppen enthalten.

Geeignete dienophile Aldehyde oder Ketone, wie sie zur Herstellung der beanspruchten neuen Verbindungen eingesetzt werden, sind ausgewählt aus der Gruppe Acrolein, Crotonaldehyd, Ethylacrolein, Penten-3-on(2), Methyl-i-propenylketon und Ethylvinylketon.

Die Geruchsnoten der neuen Verbindungen bewegen sich zwischen holzig, grün, süß bzw. Thujon-, Tabak-, Cumarin-, Galbanum-, Fichten-, Ambra-Noten und zeichnen sich durch außergewähnliche Haftfestigkeit aus. Sie können mit anderen Riechstoffen in den verschiedensten Mengenverhältnissen zu neuen Riechstoffkompositionen gemischt werden. Im allgemeinen bewegt sich der Anteil in den Riechstoffkompositionen in den Mengen von 1 bis 50 Gewichtsprozent, bezogen auf die gesamte Komposition. Derartige Kompositionen können zur Parfümierung von kosmetischen Präparaten wie Cremes, Lotionen, Duftwässern, Aerosolen, Toilettenseifen als auch in der Extrait-Parfümerie verwendet werden. Sie können aber auch zur Geruchsverbesserung technischer Produkte wie Wasch- und Reinigungsmitteln, Weichspülern, Textilbehandlungsmitteln eingesetzt werden. Zur Parfümierung der verschiedenen Produkte werden diesen die Kompositionen im allgemeinen in Konzentrationen von 0,05 bis 2 Gewichtsprozent, bezogen auf das gesamte Produkt, zugesetzt.

Der eingangs geschilderte Reaktionsablauf zur Herstellung der neuen Verbindungen kann in folgender Weise formelmäßig dargestellt werden :

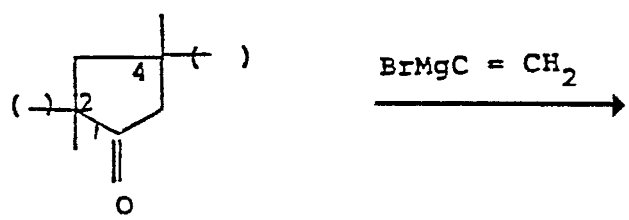


(I)

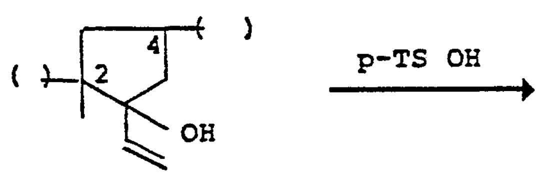


(II)

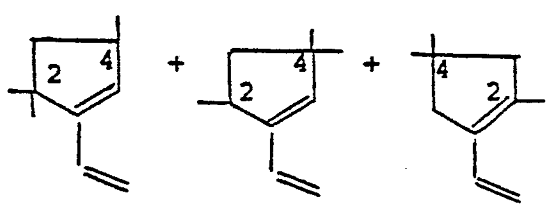

(III)

Umsetzung nach Diels-Alder mit :

Acrolein (IV)
Crotonaldehyd (V)
Ethylacrolein (VI)

Penten-3-on(2) (VII)
Methyl-i-propenylketon (VIII)
Ethylvinylketon (IX)

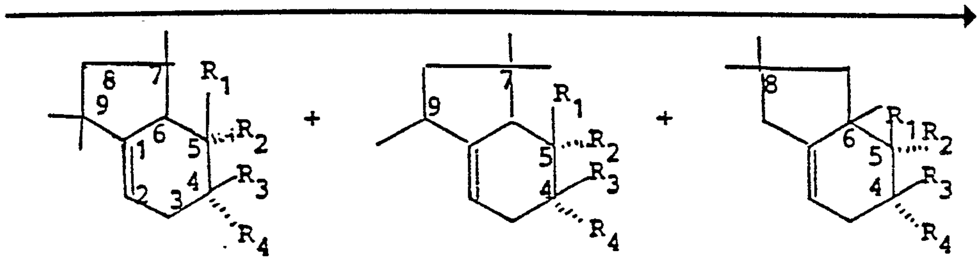

(IV-IX)

worin bedeuten :

IV. $R_1 = R_2 = H$ ; $R_3 = H$ (CHO) ; $R_4 = CHO$ (H)
$R_1 = H$ (CHO) ; $R_2 = CHO$ (H) ; $R_3 = R_4 = H$
V. $R_1 = CH_3$ (H) ; $R_2 = H$ ($CH_3$) ; $R_3 = CHO$ (H) ; $R_4 = H$ (CHO)
$R_1 = CHO$ (H) ; $R_2 = H$ (CHO) ; $R_3 = CH_3$ (H) ; $R_4 = H$ ($CH_3$)
VI. $R_1 = C_2H_5$ (CHO) ; $R_2 = CHO$ ($C_2H_5$) ; $R_3 = R_4 = H$
$R_1 = R_2 = H$ ; $R_3 = C_2H_5$ (CHO) ; $R_4 = CHO$ ($C_2H_5$)
VII. $R_1 = CH_3$ (H) ; $R_2 = H$ ($CH_3$) ; $R_3 = CH_3CO$ (H) ; $R_4 = H$ ($CH_3CO$)
$R_1 = CH_3CO$ (H) ; $R_2 = H$ ($CH_3CO$) ; $R_3 = CH_3$ (H) ; $R_4 = H$ ($CH_3$)
VIII. $R_1 = CH_3$ ($CH_3CO$) ; $R_2 = CH_3CO$ ($CH_3$) ; $R_3 = R_4 = H$
$R_1 = R_2 = H$ ; $R_3 = CH_3$ ($CH_3CO$) ; $R_4 = CH_3CO$ ($CH_3$)
IX. $R_1 = R_2 = H$ ; $R_3 = H$ ($C_2H_5CO$) ; $R_4 = C_2H_5CO$ (H)
$R_1 = H$ ($C_2H_5CO$) ; $R_2 = C_2H_5CO$ (H) ; $R_3 = R_4 = H$

Die neuen Riechstoffe stellen demnach ein Gemisch aus jeweils 3 Isomeren dar, wobei Acetyl- bzw. Aldehydgruppen und Ringverknüpfung ($C^6/C^7$)-axiale oder äquatoriale Konfiguration einnehmen können.

## Beispiele

Allgemeine Arbeitsvorschrift für die Diels-Alder-Synthese

0,1 Mol Dien (III) werden mit 0,1-0,12 Mol Dienophil (IV-IX) unter Rühren 4-9 Stunden am Rückfluß bzw. im Autoklav bei 200 °C erhitzt (im Fall von Methyl-isopropenyl-keton (VIII) unter Stickstoff bei 100 °C).

Nach beendeter Reaktion werden nicht umgesetztes Dien bzw. Dienophil abdestilliert und das Diels-Alder-Addukt im Ölpumpenvakuum fraktioniert.

1) 7,9,9(7,7,9/6,8,8)-Trimethylbicyclo [4.3.0] non-1-en-4e/a(5e/a)-carbaldehyd (IV)

Umsetzung von (III) mit Acrolein im Autoklav, 4 Stunden bei 200 °C. Kp 84-88 °C/0,6 mbar ; GC : Isomerengemisch
IR (Film) : 2 710, 1 725/cm (CHO)

| $C_{13}H_{20}O$ | Ber. | Gef. | |
|---|---|---|---|
| MG (GC/MS) | 192,3 | 192 | (Isomere) |
| % C | 80,1 | 81,2 | |
| % H | 10,5 | 10,4 | |
| % O | 8,3 | 9,5 | |

Geruch : holzig, Thujon-, Campher-Note

2) 7,9,9(7,7,9/6,8,8)-Trimethylbicyclo [4.3.0] non-1-en-4e/a(5e/a)-methyl, 4e/a(5e/a)-carbaldehyd (V)

Umsetzung von (III) mit Crotonaldehyd im Autoklav, 6 Stunden bei 200 °C,
Kp 125-135 °C C/18,6 mbar ; Isomerengemisch
IR (Film) : 2700, 1725/cm (CHO)

(Siehe Tabelle Seite 4 f.)

| $C_{14}H_{22}O$ | Ber. | Gef. | |
|---|---|---|---|
| MG (GC/MS) | 206,3 | 206 | (Isomere) |
| % C | 81,5 | 81,4 | |
| % H | 10,75 | 10,90 | |
| % O | 7,75 | 7,79 | |

Geruch : holzig, Stroh-Note

3) 7,9,9(7,7,9/6,8,8)-Trimethylbicyclo [4.3.0] non-1-en-4e/a-ethyl, 4a/e-carbaldehyd (5e/a-ethyl, 5a/e-carbaldehyd) (VI)

Umsetzung von (III) mit Ethylacrolein im Autoklav, 6 Stunden bei 200 °C.
Kp 105-115 °C/2 mbar ; GC : Isomerengemisch
IR (Film) : 2700, 1723/cm (CHO)

| $C_{15}H_{24}O$ | Ber. | Gef. | |
|---|---|---|---|
| MG (GC/MS) | 220,4 | 220 | (Isomere) |
| % C | 81,76 | 80,90 | |
| % H | 10,98 | 10,60 | |
| % O | 7,26 | 8,30 | |

Geruch : Tabak-, Cumarin-Note

4) 4e/a(5e/a)-Acetyl-4a/e(5a/e)-methyl, 7,7,9(7,9,9/6,8,8)-trimethylbicyclo [4.3.0] non-1-en (VII)

Umsetzung von I mit Penten-3-on-(2) im Autoklav, 9 Stunden bei 200 °C.
Kp 95-105 °C/2,4 mbar ; GC : Isomerengemisch
IR (Film) : 1710/cm (C = O) ; 1151/cm ($COCH_3$)
1350-1375/cm (gem. di-Methyl, $COCH_3$)

| $C_{15}H_{24}O$ | Ber. | Gef. | |
|---|---|---|---|
| MG (GC/MS) | 220,4 | 220 | (Isomere) |
| % C | 81,76 | 81,25 | |
| % H | 10,98 | 10,80 | |
| % O | 7,26 | 8,42 | |

Geruch : holzig, süß, ambriert, warm
Fichten-, Allyljonon-Note

5) 4e/a(5e/a)-Acetyl-4a/e(5a/e)-methyl, 7,9,9(7,7,9/6,8,8)-trimethylbicyclo [4.3.0] non-1-en (VIII)

Umsetzung von (III) mit Methyl-i-propenylketon bei 100 °C, 4 Stunden Rückfluß ($N_2$-Atmosphäre)
Kp 81-87 °C/0,3 mbar ; GC : Isomerengemisch
IR (Film) : 1708/cm (C = O) ; 1153/cm ($COCH_3$) ;
1350-1380 (gem. di-Methyl, $COCH_3$)
3030, 810/cm (>C = CH)

| $C_{15}H_{24}O$ | Ber. | Gef. | |
|---|---|---|---|
| MG (GC/MS) | 220,4 | 220 | (Isomere) |
| % C | 81,76 | 81,80 | |
| % H | 10,98 | 11,00 | |
| % O | 7,26 | 7,30 | |

Geruch : Galbanum-, Dipenten-Note

6) 4e/a(5e/a)-Propionyl-7,9,9(7,7,9/6,8,8)-trimethylbicyclo [4.3.0] non-1-en (IX)

Umsetzung von (III) mit Ethylvinylketon bei 170 °C, 2 Stunden Rückfluß.
Kp 105-115 °C/4 mbar ; GC : Isomerengemisch
IR (Film) : 1710/cm (C = O) ; 3020, 1340, 805 (>C = CH)

| $C_{15}H_{24}O$ | Ber. | Gef. | |
|---|---|---|---|
| MG (GC/MS) | 220,4 | 220 | (Isomere) |
| % C | 81,76 | 80,80 | |
| % H | 10,98 | 11,30 | |
| % O | 7,26 | 8,35 | |

Geruch : grün, grüne Bohnenschoten-Note

7) Kompositionsbeispiele

a) Holzbase :

| | |
|---|---|
| 4e/a(5e/a)-Acetyl-4e/a(5a/e)-methyl, 7,7,9(7,9,9/6,8,8)-trimethylbicyclo [4.3.0] non-1-en (VII) | 300 Gew.-Teile |
| 13-Oxabicyclo [10.3.0] pentadecan | 250 Gew.-Teile |
| Vetiverylacetat | 150 Gew.-Teile |
| Sandela® (Givaudan) | 150 Gew.-Teile |
| Patchouliöl | 50 Gew.-Teile |
| Ketonmoschus | 30 Gew.-Teile |
| Eichenmoos Absolue | 20 Gew.-Teile |
| Labdanum Resin | 20 Gew.-Teile |
| Myrrhe Resin | 10 Gew.-Teile |
| Pfefferöl | 5 Gew.-Teile |
| Muskatnusöl | 5 Gew.-Teile |
| Cistusöl | 5 Gew.-Teile |
| Eugenol | 5 Gew.-Teile |
| | 1 000 Gew.-Teile |

b) Fougere-Parfümierung

| | |
|---|---|
| 7,7,9(7,9,9/6,8,8)-Trimethylbicyclo [4.3.0] non-1-en-4e/a-ethyl, 4a,e-carbaldehyd (5e/a-ethyl,5a/e-carbaldehyd) (VI) | 50 Gew.-Teile |
| Formaldehyd-cyclododecyl-ethylacetal | 150 Gew.-Teile |
| Bergamottöl | 150 Gew.-Teile |
| Methyl-cyclooctylacarbonat | 100 Gew.-Teile |
| Methyljonon | 80 Gew.-Teile |
| Lavandinöl | 80 Gew.-Teile |
| Citronellol | 50 Gew.-Teile |
| Citral | 40 Gew.-Teile |
| Ambrettmoschus | 40 Gew.-Teile |
| Eichenmoos Absolue | 40 Gew.-Teile |
| Rosmarinöl | 30 Gew.-Teile |
| Orangenöl | 30 Gew.-Teile |
| Ylangöl | 30 Gew.-Teile |
| Vetiveröl | 20 Gew.-Teile |
| Lavandin Absolue | 20 Gew.-Teile |
| Benzylisoeugenol | 15 Gew.-Teile |
| Zimtblätteröl | 15 Gew.-Teile |
| Corianderöl | 15 Gew.-Teile |
| Labdanum Absolue | 15 Gew.-Teile |
| Eugenol | 10 Gew.-Teile |
| Patchouliöl | 10 Gew.-Teile |
| Thymianöl | 10 Gew.-Teile |
| | 1 000 Gew.-Teile |

**Patentansprüche**

1. 7,9,9(7,7,9/6,8,8)-Trimethylbicyclo [4.3.0] non-1-en-4e/a(5e/a)-carbaldehyd (IV).

2. 7,9,9(7,7,9/6,8,8)-Trimethylbicyclo [4.3.0] non-1-en-4e/a(5e/a)-methyl, 4e/a(5e/a)-carbaldehyd (V).

3. 7,9,9(7,7,9/6,8,8)-Trimethylbicyclo [4.3.0] non-1-en-4e/a-ethyl, 4a/e-carbaldehyd(5e/a-ethyl, 5a/e-carbaldehyd) (VI).

4. 4e/a(5e/a)-Acetyl-4a/e(5a/e)-methyl, 7,7,9(7,9,9/6,8,8)-trimethylbicyclo [4.3.0] non-1-en (VIII).

5. 4e/a(5e/a)-Acetyl-4a/e(5a/e)-methyl, 7,9,9(7,7,9/6,8,8)-trimethylbicyclo [4.3.0] non-1-en (VIII).

6. 4e/a(5e/a)-Propionyl-7,9,9(7,7,9/6,8,8)-trimethylbicyclo [4.3.0] non-1-en (IX).

7. Verfahren zur Herstellung der Trimethylbicyclo [4.3.0] non-1-en-Derivate der Ansprüche 1-6 durch Umsetzen in erster Stufe von 2,2,4(2,4,4)-Trimethylcyclopentanon(I) nach Grignard mit Vinylmagnesiumbromid zum 1-Vinyl-1-hydroxy-2,2,4(2,4,4)-trimethylcyclopentan(II), dessen Dehydratisierung in zweiter Stufe zum 1-Vinyl-2,2,4(2,4,4)-trimethylcyclo-pent-1-en (III) und weitere Umsetzung des (III) in dritter Stufe mit einem dienophilen Aldehyd oder Keton, ausgewählt aus der Gruppe Acrolein, Crotonaldehyd, Ethylacrolein, Penten-3-on(2), Methyl-i-propenylketon und Ethylvinylketon zu den Trimethylbicyclo [4.3.0] non-1-en-Derivaten der Ansprüche 1-6.

8. Verwendung der Trimethylbicyclo [4.3.0] non-1-en-Derivate des Ansprüche 1-6 als Riechstoffe.

9. Riechstoffkompositionen, gekennzeichnet durch einen Gehalt an Trimethylbicyclo [4.3.0] non-1-en-Derivaten der Ansprüche 1-6 in einer Menge von 1-50 Gewichtsprozent, bezogen auf die gesamte Komposition.

**Claims**

1. 7,9,9(7,7,9/6,8,8)-trimethylbicyclo-[4.3.0]-non-1-ene-4e/a(5e/a)-carbaldehyde (IV)
2. 7,9,9(7,7,9/6,8,8)-trimethylbicyclo-[4.3.0]-non-1-ene-4e/a(5e/a)-methyl, 4e/a(5e/a)-carbaldehyde (V).
3. 7,9,9(7,7,9/6,8,8)-trimethylbicyclo-[4.3.0]-non-1-ene-4e/a-ethyl, 4a/e-carbaldehyde(5e/a-ethyl, 5a/e-carbaldehyde) (VI).
4. 4e/a(5e/a)-acetyl-4a/e(5a/e)-methyl, 7,7,9(7,9,9/6,8,8)-trimethylbicyclo-[4.3.0]-non-1-ene (VII).
5. 4e/a(5e/a)-acetyl-4a/e(5a/e)-methyl, 7,9,9(7,7,9/6,8,8)-trimethylbicyclo-[4.3.0]-non-1-ene (VIII).
6. 4e/a(5e/a)-propionyl-7,9,9(7,7,9/6,8,8)-trimethylbicyclo-[4.3.0]-non-1-ene (IX).
7. A process for producing the trimethylbicyclo-[4.3.0]-non-1-ene derivatives claimed in Claims 1 to 6 by subjecting 2,2,4(2,4,4-trimethylcyclopentanone (I) in a first step to a Grignard reaction with vinyl magnesium bromide to form 1-vinyl-1-hydroxy-2,2,4(2,4,4)-trimethyl cyclopentane (II), dehydrating (II) in a second step to form 1-vinyl-2,2,4-(2,4,4)-trimethyl cyclopent-1-ene (III) and further reacting (III) in a third step with a dienophilic aldehyde or ketone selected from the group comprising acrolein, croton aldehyde, ethyl acrolein, penten-3-one(2), methyl-i-propenyl ketone and ethyl vinyl ketone to form the trimethylbicyclo-[4.3.0]-non-1-ene derivatives claimed in Claims 1 to 6.
8. The use of the trimethylbicyclo-[4.3.0]-non-1-ene derivatives claimed in Claims 1 to 6 as perfumes.
9. Perfume compositions, characterized by a content of the trimethylbicyclo-[4.3.0]-non-1-ene derivatives claimed in Claims 1 to 6 in a quantity of from 1 to 50 % by weight, based on the composition as a whole.

**Revendications**

1. 7,9,9(7,7,9/6,8,8)-triméthylbicyclo [4.3.0] non-1-ène-4e/a(5e/a)-carbaldéhyde (IV).
2. 7,9,9(7,7,9/6,8,8)-triméthylbicyclo [4.3.0] non-1-ène-4e/a(5e/a)-méthyle, 4e/(5e/a)-carbaldéhyde (V).
3. 7,9,9(7,7,9/6,8,8)-triméthylbicyclo [4.3.0] non-1-ène-4e/a-éthyl, 4a/e-carbaldéhyde (5e/a-éthyl, 5a/e-carbaldéhyde) (VI).
4. 4e/a(5e/a)-acétyl-4a/e(5a/e) méthyle, 7,7,9(7,9,9/6,8,8)-triméthylbicyclo [4.3.0] non-1-ène (VII).
5. 4e/a(5e/a)-acétyl-4a/e(5a/e)-méthyle, 7,9,9(7,7,9/6,8,8)-triméthylbicyclo [4.3.0] non-1-ène (VIII).
6. 4e/a(5e/a)-propionyl-7,9,9(7,7,9/6,8,8)-triméthylbicyclo [4.3.0] non-1-ène (IX).
7. Procédé pour la fabrication de dérivés triméthylbicyclo [4.3.0] non-1-ène des revendications 1 à 6, par réaction, dans une première étape, de 2,2,4(2,4,4)-triméthylcyclopentanone (I), suivant Grignard, avec du bromure de vinylmagnésium pour former le 1-vinyl-1-hydroxy-2,2,4(2,4,4-triméthylcyclopentane (II), sa déshydratation, dans une deuxième étape, en 1-vinyl-2,2,4(2,2,4)-triméthylcyclo-pent-1-ène (III), et réaction complémentaire de (III), dans une troisième étape, avec un aldéhyde ou une cétone diènophile, choisi dans le groupe des acroléine, aldéhyde crotonique, éthylacroléine, pentène-3-one (2), méthyl-i-propénylcétone, et éthylvinylcétone pour former les dérivés triméthylbicyclo [4.3.0] non-1-ène des revendications 1 à 6.
8. Utilisation des dérivés des triméthylbicyclo [4.3.0] non-1-ène des revendications 1 à 6 comme substances odorantes.
9. Compositions odorantes, caractérisées en ce qu'elles contiennent des dérivés du triméthylbicyclo [4.3.0] non-1-ène des revendications 1 à 6, dans une proportion de 1 à 50 % en poids, calculé sur le total de la composition.